(19) **Europäisches Patentamt**
**European Patent Office**
**Office européen des brevets**

(11) **EP 4 729 982 A1**

(12) **EUROPEAN PATENT APPLICATION**

(43) Date of publication:
**22.04.2026 Bulletin 2026/17**

(21) Application number: **25208992.5**

(22) Date of filing: **15.10.2025**

(51) International Patent Classification (IPC):
**G01S 7/52** *(2006.01)*    **G01S 15/89** *(2006.01)*
**G10K 11/34** *(2006.01)*    **A61B 8/00** *(2006.01)*

(52) Cooperative Patent Classification (CPC):
**G01S 15/8915; A61B 8/4472; A61B 8/5207;
A61B 8/565; G01S 7/52034; G10K 11/341;
A61B 8/488**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **17.10.2024 EP 24207345**

(71) Applicant: **ECOLE POLYTECHNIQUE FEDERALE
DE LAUSANNE
(EPFL)
1015 Lausanne (CH)**

(72) Inventors:
• **Viñals Terres, Roser**
**1015 Lausanne (CH)**
• **Thiran, Jean-Philippe**
**1015 Lausanne (CH)**

(74) Representative: **Fleuchaus & Gallo Partnerschaft
mbB
Steinerstraße 15/A
81369 München (DE)**

(54) **ULTRASOUND IMAGING WITH BEAMFORMING FROM DATA THAT ARE ENHANCED BY A NEURAL NETWORK IN THE SIGNAL DOMAIN**

(57)    To obtain a data-structure that is characteristic of a physical object, a computer receives (413) sensor-data ($\{\{Y\}\}\downarrow$) from a sensor-array. The received sensor-data ($\{\{Y\}\}\downarrow$) is - in spatial and temporal data arrangement - a sub-set of sensor-data of a reference sensor-array. The computer uses a neural network to enhance (433) the received sensor-data to enhanced sensor-data so that - in spatial and temporal data arrangement - the enhanced sensor-data correspond to the sensor-data of the reference sensor-array. An imaging module beamforms (453) the enhanced sensor-data to the data-structure. The neural network has been trained (402) to obtain network weights, with sensor-data from the ultrasound transducer elements of the reference sensor-array.

FIG. 8

EP 4 729 982 A1

## Description

## Technical Field

The disclosure generally relates to digital signal processing, and more in particular, it describes a computer-implemented method as well as a computer to obtain an ultrasound data-structures, such as images from physical objects, with signal preprocessing by a neural network and by beamforming.

## Background

Much simplified, imaging techniques enable computers to represent properties of physical objects by image data in such a way that computer users can easily understand the properties.

Sensor-arrays comprise multiple sensor-elements, and each sensor-element provides an electrical signal. Converting electrical signals to computer-readable data is implemented by appropriate converters (i.e., analog-to-digital converters or the like), that are usually integrated into the arrays. The data at the output of the converters is called "sensor-data" or "raw data". The sensor-arrays are usually integrated into devices that comprise further elements, such as housings, user interfaces, storage, communication interfaces or the like.

The computer processes the sensor-data such that the properties of the physical object are visualized in an image. While images with pixels in two Cartesian coordinates (2D) are standard in the art, there is a trend to provide images with further dimensions (such as 3D-images, with 3D-pixels, so-called voxels etc.).

For example, the computer obtains the data from the optical sensor-array of a camera device. The sensor-elements of the sensor-array are sensitive to visible light and they catch the color at different surface locations of the object. In a variation, a thermal sensor-array provides data that correspond to the location-specific infrared radiation of the object surface, and the computer provides the image as a so-called heat-map.

Ultrasound sensors comprise (ultrasound) arrays with ultrasound sensor-elements. Usually, the ultrasound sensor-elements are implemented as transducer elements that (i) emit ultrasound pulses and (ii) measure the echo returned by the object (such as by the object surface or by internal structures within the object). The signals from each transducer element are analog-to-digital converted to sensor-data as well.

Processing the sensor-data to images takes the particulars of sound propagation into account. For example, the echoes of a single ultrasound pulse would hit multiple transducer elements (usually with different strength and with different delay). As the sensor-data would change at a relatively high frequency (with frame rates of multiple kilohertz), the echoes are also attributed to be radio frequency (RF) signals. Techniques to process the sensor-data to images are called beamforming, and a prominent beamforming approach uses "delay and sum (DAS)".

Convenient references to consult are:

- R. Cohen and Y. C. Eldar, "Sparse convolutional beamforming for ultrasound imaging", IEEE Transactions on Ultrasonics, Ferroelectrics, and Frequency Control, vol. 65, no. 12, 2018.

- Roser Viñals and Jean-Philippe Thiran, "A KL Divergence-Based Loss for In Vivo Ultrafast Ultrasound Image Enhancement with Deep Learning", DOI: 10.36227/techrxiv.23939970.v1, the Viñals/Thiran paper in short.

There are multiple constraints that show up in combination, such as in the following examples:

- A delay occurs from data acquisition by the sensor-array and transmitting data to the computer, to providing the image by the computer. But for real-time applications, delays need to be avoided.

- There can be limitations in the resolution (i.e., granularity in space).

- In some applications, the movement of the object has to be monitored but data changes more frequently in comparison to static objects.

In principle, these multiple constraints are related to image processing in general, but for ultrasound imaging the constraints seem to be more severe.

For many applications, the time from operating the sensor-array to providing the image to a human computer user should be as short as possible. This time constraint is important, for example, because the computer user would need to interact with the object in some way in real-time.

Sensor-arrays comprise elements in a number that allows imaging in an accuracy (in terms of spatial resolution, contrast in the images etc.) that corresponds to the application. While technological process in the last decades allowed to manufacture optical sensors with millions of elements, increasing the element number in ultrasound arrays is technically more challenging.

There is a trend to communicatively connect ultrasound sensor-arrays to the computers by wireless connections. The ultrasound sensor-arrays are integrated into ultrasound devices with wireless communication interfaces. But in comparison to cable connections, wireless connections have less bandwidth.

For medical applications, the ultrasound devices are also called ultrasound probes. A body region of a patient is the object, and a medical practitioner is the computer user. Ultrasound imaging serves as a diagnostic tool, due to its real-time ability to produce high-quality images of soft tissues in the body. Both the patient and practitioner are usually interested in the movement of the body. For example, they need to know if an organ moves, and how it does. In that sense, the images turn into a video that need to be captured in real-time.

Ultrasound probes tend to have wireless communication interfaces, but real-time requirements may not be compatible with bandwidth limitations that such wireless interfaces may have.

The skilled person would try mitigating these and other of the mentioned multiple constraints by switching to more sophisticated hardware: faster processing, more elements, using a wireless connection with relatively high bandwidth, processing data in parallel and so on. However, this is not possible for all applications.

In contrast to light imaging or heat radiation imaging, ultrasound imaging requires the application of energy pulses (from the transducers), and there is a trend to apply unfocused wavefronts that disperse energy across the entire field of observation (cf. the Viñals/Thiran paper)

### Summary

The sensor-arrays may provide data as a data selection. The data selection can be optimized in view of available bandwidth for transmitting data to the beamforming computer. There are - in principle - two options to select data:

- In a so-called "sparse array" approach, the sensor-array that has N|f elements and can provide data from a sub-set N|s of the N|f elements.

- In a down-sampling approach, the sensor-array can provide data from selected time-slots only.

Both data selection options can be combined.

To obtain a data-structure (such as for example, an ultrasound image) that is still characteristic of physical object - despite the data-selection - a computer receives sensor-data from the sensor-array. The received sensor-data is - in spatial and temporal data arrangement - a sub-set of sensor-data of a reference sensor-array. The computer uses a neural network to enhance the received sensor-data to enhanced sensor-data so that - in spatial and temporal data arrangement - the enhanced sensor-data correspond to the sensor-data of the reference sensor-array. This enables beamforming. An imaging module beamforms the enhanced sensor-data to the data-structure. More in general, the imaging module generates the data-structure from the enhanced sensor-data. The neural network has been trained to obtain the network weights, with sensor-data from the ultrasound transducer elements of the reference sensor-array.

As disclosed herein, a computer-implemented method has the purpose to obtain a data-structure that is characteristic of a physical object. The method involves beamforming. A computer receives sensor-data from a sensor-array. The received sensor-data is - in spatial and temporal data arrangement - a sub-set of sensor-data of a reference sensor-array that has N|f ultrasound sensor-elements. A neural network of the computer uses weights. The network enhances the received sensor-data to enhanced sensor-data so that - in spatial and temporal data arrangement - the enhanced sensor-data correspond to the sensor-data of the reference sensor-array. An imaging module of the computer beamforms the enhanced sensor-data to the data-structure. The neural network has been trained to obtain the network weights, with sensor-data from N|f > N|s ultrasound transducer elements of the reference sensor-array with N|f ultrasound sensor-elements.

Optionally, the computer performs the enhancing step such that the received sensor-data go to an input of the neural network and the enhanced sensor-data are available at an output of the neural network.

Optionally, the neural network has been trained to obtain the network weights, with the neural network under training having received the sensor-data from the reference sensor-array at the output, and having received selected sensor-data from N|s < N|f ultrasound transducer elements from the reference sensor-array through a selector module that applies a selection.

Optionally, in the step receiving, the received sensor-data is a sub-set of sensor-data of a reference sensor-array that is any of the following: (i) a sub-set of sensor-data that is based on a spatial selection that identifies a subset of sensor-elements for that data is to be communicated to the computer, (ii) a sub-set of sensor-data that is based on a temporal selection that identifies a subset of time-slots for that data is to be communicated to the computer, or (iii) a sub-set of sensor-data that is based on the combination of the spatial selection and the temporal selection.

Optionally, the selector module applies the selection by the following: the selector module performs the receiving step to receive the sensor-data from the N|f > N|s ultrasound transducer elements of the reference sensor-array, and the selector module applies a selection matrix function.

Optionally, in the step enhancing, the computer provides a delta data pattern and in a following step combining, the computer combines the delta data pattern to the received sensor-data.

Optionally, the sensor-data from the sensor-array with N|s sensor-elements and the sensor-data from the reference sensor-array is data in a signal-domain that is represented as multi-variate time-series with the number of variates corresponding to the number of sensor-elements that provide data, and with a number of time-slots for sample-measuring an echo following a wavefront generation.

Optionally, the step enhancing is performed by a network that has a deep learning architecture.

Optionally, wherein the data-structure is any of the following: a speed-of-sound image, a Doppler image, and an ultrasound image.

Optionally, the neural network has been trained in advance with sensor-data from N|f > N|s ultrasound sensor-elements of the reference sensor-array at the output and with selected sensor-data at the input.

Optionally, the neural network has been trained with calculating a loss function in the signal-domain with multi-variate time-series of the reference sensor-array as the ground truth.

Optionally, the neural network has been trained with further calculating a loss function in an image-domain by beamforming intermediate data to hidden images that are compared to the ground truth of beamformed full-number images.

Optionally, to obtain the network weights during training - both the loss function in the signal-domain and the loss-function in the image-domain are combined such that the loss functions have different loss weights.

As disclosed, a computer system is adapted to obtain a data-structure that is characteristic of a physical object. The computer system is therefore adapted to execute the computer-implemented method.

A computer program product that - when loaded into a memory of a computer and being executed by at least one processor of the computer - causes the computer to perform the steps of the computer-implemented method.

## Brief Description of the Drawings

FIG. 1 gives a simplified overview to ultrasound imaging, by way of example for an application and for a use-case scenario;

FIG. 2 illustrates a linear sensor-array with N sensor-elements in linear arrangement and illustrates a two-dimensional sensor-array with sensors in rows and columns;

FIG. 3 illustrates data in relation to sensor-elements, being data that is communicated from the sensor-array to the computer;

FIG. 4 illustrates an overview to network phases;

FIGS. 5A and 5B illustrate ultrasound imaging, from left to right, with a physical object, ultrasound waves, a sensor-array, time-series, and an ultrasound data-structure;

FIG. 6 illustrates a block-diagram of a system to obtain an ultrasound data-structure of a physical object by beamforming, wherein the system comprises a computer with modules to perform a computer-implemented method to obtain the data-structure of the physical object by beamforming;

FIG. 8 illustrates flow-charts for a computer-implemented method to obtain the ultrasound data-structure, and for a computer-implemented method to train the network;

FIG. 9 illustrates a settings configuration module as well as a method for training and a method for monitoring with the steps for that the settings can be relevant;

FIG. 10 illustrates a generic computer.

## Detailed Description

### Introduction

The description explains a method to obtain a data-structure that is characteristic of a physical object and that is obtained by sonography. In other words, the data-structure is a sonographic data-structure. The method involves beamforming data from ultrasound sensors. For many applications, the data-structure can be visualized to a human user, and the description therefore uses the term "image" (for the data-structure). However, it may not be required to present an image on a display. The data-structure could be post-processed by a further computer so that human users can learn properties of the object.

As sound techniques are being applied (especially ultrasound techniques), the data-structure describes acoustical properties of the object. The data-structure can represent, for example,

- differences in the sound propagation speed within the object ("speed-of-sound image"),

- differences in sound frequencies for objects (or parts of the objects) that are moving ("Doppler image", data obtained by Doppler ultrasonography),

- differences in reflections and other interactions of sound and object ("ultrasound image").

As the skilled person is familiar with acoustical imaging techniques, the description takes ultrasound imaging as a representative technology.

### Application example

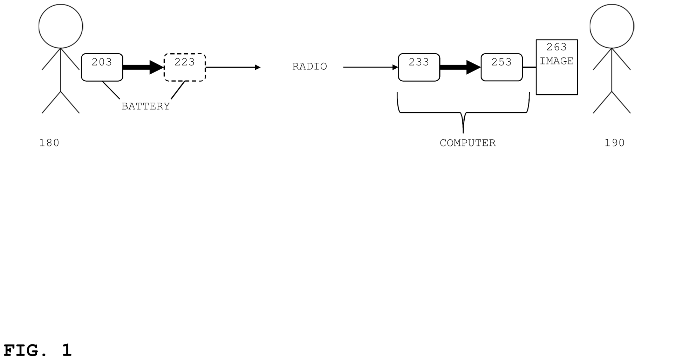

FIG. 1 gives a simplified overview to ultrasound imaging, by way of example for an application and for a use-case scenario.

As used herein, the term "medical application" refers to the above-mentioned situation in that a medical practitioner 193 is the computer user who investigates the properties of a particular region of a human body (i.e.

of patient 183). The body region is an example of an "object under observation". FIG. 1 also shows the data-structure as image 263. Medical practitioner 193 would see from image 263 that, for example, a bone is broken. Further computers that process that data-structure may come to the same result, but without presenting an image.

For some aspects of the medical application, it is contemplated to have patient 183 and practitioner 193 located remotely to each other (i.e., the practitioner can't see the patient directly). Investigating remotely inherently requires to transmit data, but bandwidth restrictions can become a constraint.

Describing a medical example is convenient for explanation, but not limiting. Data processing (that belongs to the methods) is not specific to such an application. The skilled person can therefore modify the application, for example, to let the computer observe a non-human object (e.g., user being a veterinarian who investigates the health of an animal).

Use-case scenarios describe circumstances of the application's performance. For example, in a wireless scenario (with a radio link RADIO), ultrasound sensor-array 203 (that obtains data from the object, i.e., from patient 183) communicates with the computer (that processes the data to the image, here with computer modules 233/253) via a bandwidth-limited data connection (thin arrows). Further, the device (that comprises the ultrasound sensor-array 203) would be portable (or even "ultra-portable"), and the electrical energy for sensing and for transmitting data would have to come from a battery.

A variation of the wireless scenario is the mobile computer scenario, in that the processing would be performed by computers that comprise a portable computer, such as a laptop computer, or a mobile telecommunication device (i.e., a Smartphone or the like). Such computers are usually not designed to connect to such wireless ultrasound devices.

Even worse, the medical application in a wireless scenario (i.e., the RADIO link) would create a constraint conflict: The practitioner eventually needs to interact with the patient (here practitioner 193 and patient 183). Images should be provided in real-time (i.e., that means without significant delay), but data transmission from the ultrasound probe over radio (i.e., wireless) might not provide sufficient bandwidth.

Real-time requirements are related to the interaction between the practitioner and the patient. If something is wrong with the patient, the practitioner needs to know that immediately, simply to provide appropriate measures.

In a first example, if a particular organ changes its movement pattern, that change should be visible on the computer display without delay. In a second example, the medical practitioner applies biopsy techniques to remove a tissue sample from the human body. The sample is to be examined under a microscope later.

The sample needs to be located first. Traditional biopsy approaches are radiation guided, but ionizing radiation (X-rays etc.) should be avoided. Ultrasound-guided approaches avoid exposure to such radiation. But any delay needs to be minimized, the sample has to be removed, nothing else.

## Overview

Ultrasound sensor-array 203 can provide data as a data selection. To be explained below, the data selection can be in space and time. In the figure, the data selection is symbolized by an arrow turning from bold to thin, and in the description, the selection is also symbolized by a ↓ symbol.

Sensor-array 203 can be associated with selector module 223 (optional part) that selects data for communicating to the image processing computer, or sensor-array 203 provides selected data originally (without the selector module). Image processing computer 233/253 can comprise neural network 233 that enhances the selection (and at least imitates the reversal of the data selection, i.e., the de-selection) and imaging module 253 that establishes an image (to be displayed to practitioner 193).

## Sensor-arrays and data selection

The description shortly defines writing conventions for sensor-arrays 200-1, 200-2 (collectively: 200) and for the data selection, here symbolized by selector modules 220-1 and 220-2 (collectively: 220).

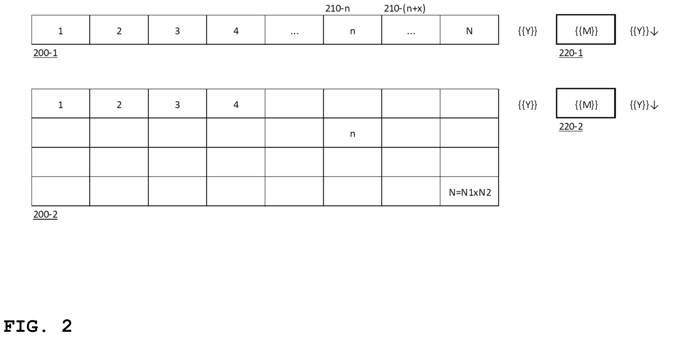

FIG. 2 illustrates a linear sensor-array 200-1 with N sensor-elements in linear arrangement and illustrates a 2D sensor-array 200-2 with N = N1 * N2 sensor-elements in N1 rows and N2 columns. The skilled person is familiar with arranging sensor-elements linearly (as in 200-1), in a convex curve, in an array (i.e., as in 200-2, in a matrix), or otherwise.

Index n is a sensor-element index that identifies individual sensor-elements 210-n. The skilled person can use other arrangements (e.g., arrays with further dimensions, arrays in circles or in other shapes), but the common convention is that any sensor-element 210-n is located at a different position than sensor-element 210-(n+x). In other words, each sensor-element has its own unique position in space.

Sensor-arrays 200 can have

- transmit-only elements that provide sound bursts (or so-called wavefronts) only,

- receive-only elements that receive sound echoes (from the wavefronts) only, or

- combined elements or "transducer elements" that can - at different points in time - can provide sound bursts and can receive echoes.

Although transducer elements can do both actions, some of the transducer elements may act as receive-only elements or may act as transmit-only elements. This partial non-use of functionality can be applied to situations (e.g., sparse arrays) in that transmission bandwidth is limited.

Bandwidth constraints in the data communication from the computer to the sensor-array can be neglected. The computer may trigger the sensor-array to provide sound-bursts, or may identify a selection scheme (cf. a discussion of a selection matrix, further below).

In implementations, sensor-array 200 provides the bursts periodically, with all transducer elements. Modifications are possible (e.g., using a limited number of transducer elements, changing the burst providing transducer elements, etc.).

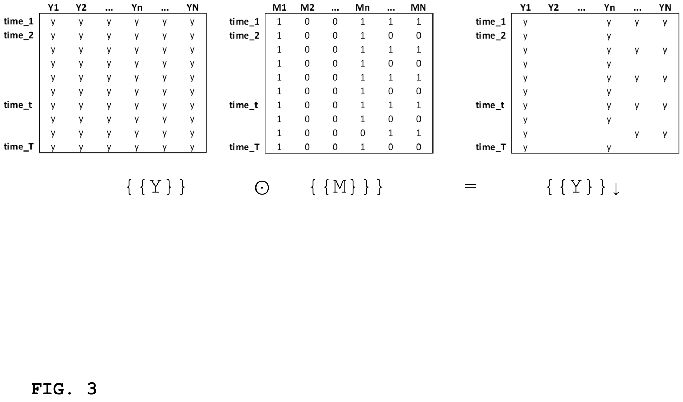

FIG. 3 illustrates data in relation to sensor-elements, being data being transmitted from the sensor-array to the computer. The data from the sensor-elements are shown as a multi-variate time-series $\{\{Y\}\}$ that represents the received echo.

• N individual sensor-elements with index n are the "multi-variates" given here in columns.

• The progress of time is represented by T sampling time-slots time_t with index t, from time_1 to time_T. The duration of a time-slot (i.e., from time_t to time_(t+1) is the inverse of the sampling rate.

The skilled person occasionally may apply the term "channel": Data from N sensor-elements would be communicated to the computer in N channels.

The number of sensor-elements N is related to the spatial resolution of sensor-array 200 (i.e., the pitch between the elements), N is pre-defined by the manufacturer of the sensor-arrays (and of the ultrasound devices), and the user can not change N.

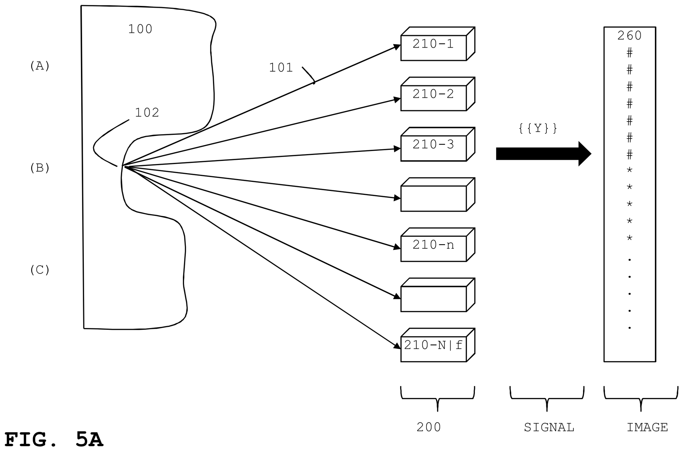

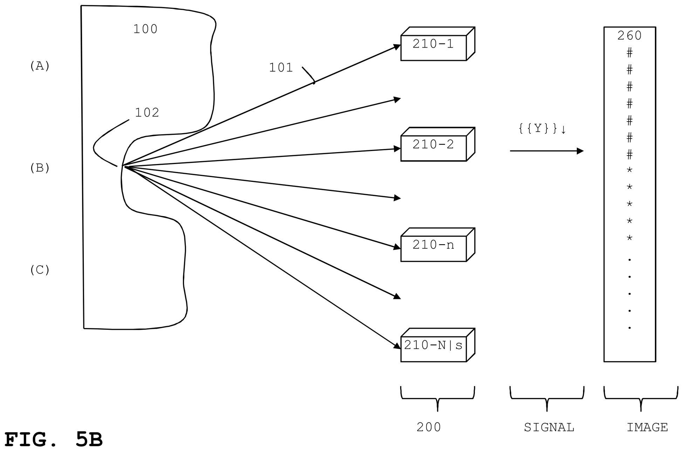

The number of time-slots T is related to the spatial distance (or object depth) from that echoes are expected, in relation to the wave propagation speed inside the object (i.e., with higher T for larger depths). Much simplified, the object depth is the physical distance between the sensor-array and various parts of the object, cf. FIGS. 5A, 5B). The time-slot number T is selectable by the user as a data-acquisition setting, with minimal and maximal values.

As the skilled person is familiar with the selection of N and T, the description does not have to provide further explanation.

A multi-variate time-series $\{\{Y\}\}$ has N single-variate time-series Yn (from Y1 to YN, with T time-slots per time-series).

In the figure, the small "y" stands for the data values that the sensor-elements provide. In other words, $\{\{Y\}\}$ stands for the spatial and temporal data arrangement that is independent from particular values y.

The skilled person could illustrate data values by colors or otherwise (such as in "heatmaps"), but such illustration is not required here.

Assuming for example, that all N elements are transducer elements, they would transmit a single acoustical burst (an initial wavefront) and they would receive and would measure a response (i.e., sound waves) to provide $\{\{Y\}\}$. By convention, a particular multi-variate time-series $\{\{Y\}\}$ fits to a particular burst. For a new burst, a new $\{\{Y\}\}$ is obtained.

The skilled person is able to forward $\{\{Y$ to a computer that would calculate an image (cf. computer at least having imaging module 253). However, depending on use-case scenarios,

• there can be bandwidth limitations in the communication link from the sensor-array to the computer, there are many occasions in that $\{\{Y\}\}$ will not be communicated to the computer completely, or

• there can be limitations in the number of sensor-elements so that $\{\{Y\}\}$ will not be measured to obtain an image that fits to the application.

Instead of communicating the multi-variate time-series $\{\{Y\}\}$, a subset of $\{\{Y\}\}$ is selected and communicated. This subset is noted as $\{\{Y\}\}.\downarrow$.. This subset $\{\{Y\}\}\downarrow$ can be pre-defined in space, can be predefined in time or can be pre-defined in a combination of space and time.

• A spatial selection identifies a subset of sensor-elements 210-n (cf. FIG. 2) for that receive-data is to be communicated to the computer. This spatial selection will be summarized with the letter N and with indices to N, such as indices |f and |s.

• A time selection (or temporal selection) identifies a subset of time-slots for that data is to be communicated. This time selection will be summarized and explained but - for simplicity - further indices are not used.

FIG. 3 further illustrates a selection matrix $\{\{M\}\}$. In terms of rows (t) and columns (n), the selection matrix $\{\{M\}\}$ fits multi-variate time-series $\{\{Y\}\}$. For every matrix cell (n, t) in $\{\{M\}\}$, the matrix comprises an indication if data from $\{\{Y\}\}$ is selected or not:

$$\{\{Y\}\}\downarrow = \{\{M\}\} \odot \{\{Y\}\}.$$

The data in $\{\{M\}\}$ is binary data, such as "to take over" or "to ignore". For simplicity, the figure uses "1" and "0", and the $\odot$ operation can be multiplication.

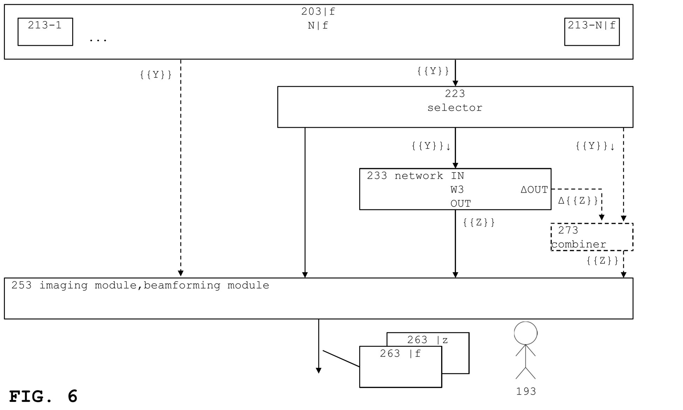

Writing $\{\{M\}\}$ is just a convenient way to formulate the selection $\downarrow$ in general, but the selector module (cf. 223 in FIGS. 1 and 6) does not have to perform a matrix operation. Data selection can be implemented otherwise.

In the example, cells with "1" stand for taking over data (matrix element wise multiplying with 1 does not change that), and cells with "0" stand for ignoring data. In the example, matrix {{M}} can be taken as a multiplier matrix (element-wise multiplication, multiplication with zero ignores the data). The skilled person is able to technically implement such a selection, but a matrix calculation is not required.

In principle, there are as many options as the 1 and 0 can be combined in the matrix, and the following selection patterns are preferred to apply:

- As a spatial selection, it is possible to take over data from every second sensor-element (selection in space, in {{M}} that would be a column with 1, a column with 0, a column with 1 and so on, cf. the below-explained notation: N|s = N|f /2). It is also possible to take over data from every third sensor-element, every fourth sensor-element, also noted as N|s = N|f /3 and N|s = N|f/4.

- As a temporal selection, it is possible to take over a pre-defined number of time-slots at the beginning (i.e., t = 1, t = 2 etc.) but from a particular time-slot (e.g., t = 3) to ignore every second time-slot (selection in time, in {{M}} that would be a change from row to row).

- As a temporal selection, it is possible to take over every second time-slots (selection in time, like down-sampling in traditional signal processing, in {{M}} a row with 1 only, a row with 0 only, a row with 1 only, and so on).

The selection patterns are examples, and combinations for selection in space and in time are possible. It is also contemplated use a random selection (the matrix elements in {{M}} would be obtained by a random generator that selects the time and/or selects the space).

**Writing convention for N**

Instead of indicating details for matrix {{M}}, the description can also use a simplified notation. As used herein, the uppercase letter N stands

- for the number of sensor-elements that belong to a sensor-array (cf. FIG. 1), and

- for the number for single-variate time-series {Y}n that such a sensor-array can provide (before any selection).

A sensor-array with N elements can provide N time-series {Y}n of sensor-data. There is a simplified assumption that the computer would process these N time-series.

For example, in implementations, the sensor-array belongs to a probe that is commercially available and that has a linear array transducer with N = 192 transducer elements (e.g., GE 9L-D probe, from GE Healthcare, Chicago, Illinois, USA).

As mentioned already, the description differentiates two kinds of sensor-arrays. With small letter "f" being the acronym for "full-number", and "s" being the acronym for "sparse", the following conventions are applied:

- A full-number sensor-array has N|f sensor-elements and can provide N|f time-series {Y}n of sensor-data. In other words, a full-number sensor array provides {{Y}}.

- A sparse sensor-array has N|s sensor-elements that provide data and provides only N|s time-series {Y}n of sensor-data. A data selection is applied to the sensor-elements (cf. M1 = 1 selects all time-slots from sensor 1, M2 = 0 selects all time-slots from sensor 2, and so on). In other words, a sparse sensor array provides {{Y}}↓ that is a sub-set (cf. the columns with 1 in {{M}}).

As already mentioned N|f is a sensor parameters that are given by the manufacturers. N|s can be defined by bandwidth limitations (e.g., RADIO in FIG. 1), with N|s < N|f.

For training the neural network, it is possible to convert data from {{Y}} to {{Y}}↓ by simply applying {{M}}. In other words, for training purposes, full-number sensor-arrays can be used, but for some training steps the full-number sensor-arrays are downgraded to behave like sparse sensor-arrays (and/or to provide data that is down-sampled).

There are no absolute thresholds to differentiate "full-number" from "sparse" arrays, the differentiation is a relative one. Going "sparse" can be advantageous, in terms of hardware requirements, bandwidth savings in transmitting signals, etc. In alternative terms, the sensor-arrays are high-number sensor-arrays or low-number sensor-arrays. As the amount of data to be communicated to imaging module 253 (cf. FIG. 1) would be larger for full-number sensor-arrays and smaller for sparse-sensor arrays, bandwidth can be saved for sparse arrays.

It is - optionally - possible to operate sensors with N|f sensor-elements in two modes:

- in full mode, to provide N|f time-series {Y}n, i.e., to provide {{Y}}, or

- in sparse mode, to provide N|s time-series {Y}n, i.e., providing {{Y}}↓, optionally with temporal selection as well.

This operation mode is symbolized by the matrix {{M}}, all "1" would stand for the full mode, and at least some "0" would stand for the sparse mode.

There is a difference, N|f > N|s, but this lack of data from sparse sensor-arrays can be compensated by appropriately training a network that enhances the data. As a result, the amount of data would reach N|f time-series (or even surpass N|f).

By introducing phases, the description will explain that - for use during training the network in phase \*\*2 - sub-sets of data (i.e., selections that can be defined), and that the trained network in phase \*\*3 provides data in an amount that corresponds to N|f.

Processing multi-variate time-series {{Y}} by matrix {{M}} can metaphorically be called "masking": some of the content in {{Y}} is being masked (in the sense of "filtered out").

**Writing convention in view of tool phases**

The description refers to computer-implemented methods that involve the use of machine learning (ML) tools or modules, such as neural networks (cf. network 233 in FIG. 1 for example). The same applies for computers that execute the methods.

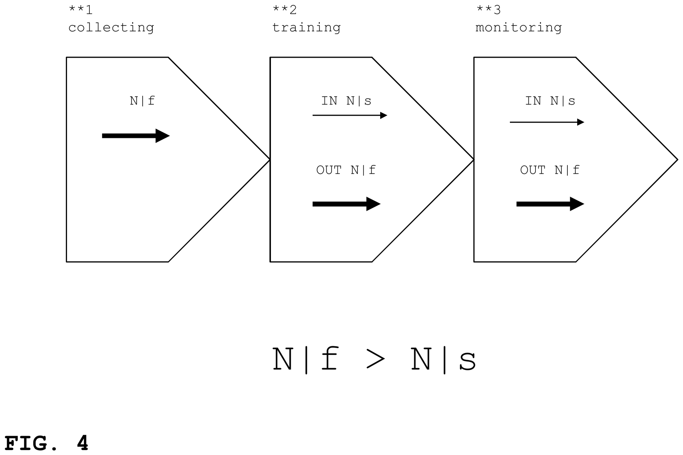

FIG. 4 illustrates an overview to network phases \*\*1, \*\*2 and \*\*3. In other words, the network is an ML-tool and operates differently when it is being trained to obtain network weights, and when it uses the network weights later on.

It is convenient to differentiate the following:

- A collecting phase \*\*1 refers to the time when the computer collects historical data to obtain a training data set for the network. During that phase \*\*1, the network does not have to operate. It even does not yet have to exist.

- A training phase \*\*2 refers to time when the computer applies the training data set to train the network (cf. method 402 in FIG. 4). The network is the "network under training". Training phase \*\*2 should not involve human experts, at least to obtain one or more ground truths (GT). As it will be explained with more detail below, the GTs are obtained from sensors in full mode (N|f, {{Y}}). Human experts are not involved. The training is therefore unsupervised.

- A monitoring phase \*\*3 refers to the time when the computer uses the trained network, in a method to obtain properties of a physical object (cf. method 403 in FIG. 4, cf. FIG. 1 for the medical application as an example). To stay with the medical application, the monitoring phase \*\*3 is also the time when patient 183 and practitioner 193 becomes involved.

The notation \*\*\* applies to the references as well. For example, reference "200" stands for a sensor-array that provides sensor-data (cf. FIGS. 1, 5A, 5B), at any time but not specific to a phase, but reference "203" stands for the sensor-array that provides data to be processed during the operation of the network when the object is being monitored (cf. FIG. 1).

Neural network 233 is a trained network (cf. FIG. 6) that participates in the execution of a method to obtain an ultrasound image (method 403, cf. FIG. 6), and neural network 232 is a network under training.

The above-mentioned constraints (delay, granularity, movement etc.) are mostly applicable during the monitoring phase \*\*3. But as it will be explained, the way the data is collected in phase \*\*1 and the way the tool is being trained in phase \*\*2 is relevant to address these constraints.

The above-mentioned constraints are less relevant for the collecting phase \*\*1. For example, in the above-shown medical application example, there would be no bandwidth restriction (and hence no constraints in view of delay, granularity, movement) because sensor-data {{Y}} can be communicated to the computer via a cable. In other words, there is plenty of time to receive and to store data (from sensor-arrays) and images do not have to be provided (and do not have to be presented to a user).

Although the phases are illustrated consecutively (here from left to right), it is possible to repeat some of them, for example to repeat training phase \*\*2 (to perform re-training) as soon as further historical data becomes available.

- In collecting phase \*\*1, data can be available as N|f multi-variate time-series {{Y}} from N |f sensor-elements, cf. {{Y}} in FIG. 3.

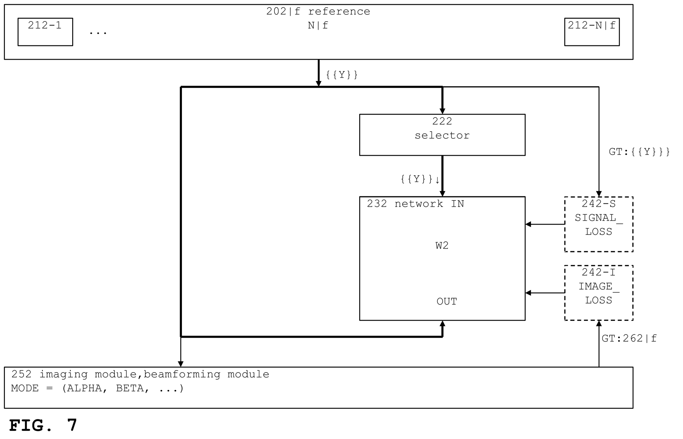

- In training phase \*\*2, cf. FIG. 7, data can be made available to a network input IN with some data values missing (cf. for example as N|s or as {{Y}}↓ in general) and can be made available to a network output OUT with full-number data (cf. {{Y}}).

- In monitoring phase \*\*3, the network had been trained to complete missing data, the input IN sees less data (cf. {{Y}}↓ in FIG. 6, but not {{Y}}) but the output OUT provides {{Z}} ≈ {{Y}}.

The phases can also be differentiated by data quantity. During collection \*\*1, multi-variate time-series {{Y}} are being collected in multiple sets. The number of time-series {{Y}} that are collected is at least the number of time-series that are to be used as reference time-series {{Y}} during training.

During monitoring \*\*3 (cf. the above application / scenario), a single time-series {Y}}↓ would be the basis for obtaining the ultrasound images (that is to be presented to the user).

**Example**

FIGS. 5A and 5B illustrate ultrasound imaging (as an example to obtain the data-structure that is characteristic for the object), with differentiating

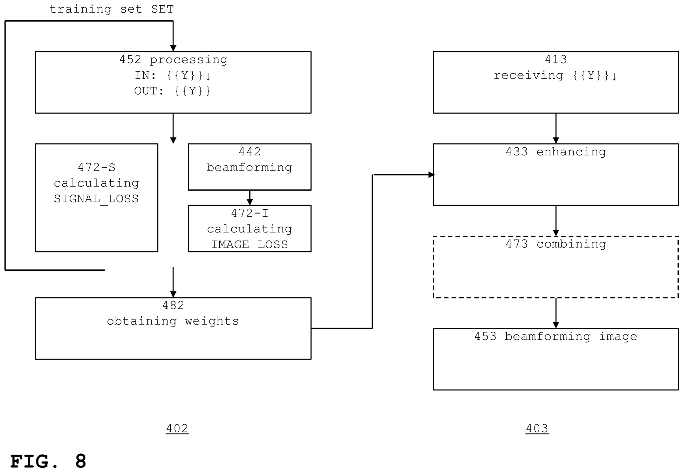

- a traditional approach in FIG. 5A, and
- an approach in FIG. 5B with a computer that performs a method that is described here (method 403 in FIG. 8, with a neural network that enhances sensor-data ({{Y}}↓).

From left to right, the figures show

- physical object 100 (that is the object under observation, that is general for the particular region of the human body, in item 183 in FIG. 1),

- back-propagated waves 101 from the object,

- sensor-array 200 to sense the back-propagated waves from the object, with sensor-elements 210-1 to 210-N (N|f in FIG. 5A and N|s in FIG. 5B).

- sensor-data as multi-variate time-series {{Y}} in FIG. 5A or {{Y}} ↓ in FIG. 5B

- the data-structure as ultrasound image 260.

Computer and communication lines to transmit data are omitted. Some of the references will be repeated in other figures.

**Object**

Different parts of object 100 reflect sound waves differently, due to properties such as, for example: density and chemical structure, liquid content (such as the share of water), or thickness.
In the example, physical object 100 is illustrated in side-view for a fictitious shape. It may move or not, but it is illustrated as not moving.

- Part (A) of the object is illustrated at the top. In relation to sensor-array 200, part (A) is located at relative low distance, so that - simplified - the echo waves arrive at the sensor with highest magnitude.

- Part (B) of the object is located in its center, but at relative high distance. Echo waves would arrive later and at weaker magnitudes. By way of example, location 102 stands for a location that is hit by a burst and that reflects echo waves, in several directions. The figure is simplified in that echo waves would hit all sensor elements. Location 102 merely serves as an example, because echo waves would come from other locations as well (e.g. from the surface as a whole).

- Part (C) of the object is illustrated below, for simplicity, the echo waves are omitted.

In the medical application, physical object 100 can be a region of a human or animal body. For simplicity, the figure does not illustrate the skin or other regions that conduct the echo waves on their way from the region to the sensor-array.

**Sensor-array**

The skilled person is familiar with integrating ultrasound sensor-arrays into devices, such as into ultrasound probes. The ultrasound devices provide data connection (i.e., the mentioned communication interface with a cable, or with a radio transmitter, but with limited bandwidth), provide a handle for the user, provide easy-to-clean surfaces and the like.
In the medical application, the sensor-array would be part of a hand-held ultrasound probe, and the practitioner would move the probe over the skin of the patient. Going wireless is a trend for medical applications as well.

**Full-number or sparse array**

In the example, sensor-array 200 can be a full-number sensor-array with N|f sensor-elements as in FIG. 5A, or can be a sparse sensor-array with N|s sensor-elements as in FIG. 5B (or bandwidth limited transmission turns "full" into "sparse"). It is noted that the echo waves would arrive in any case, no matter how many sensor-elements the sensor-array has. This is symbolized in FIG. 5B by some lines for waves 101 not leading to sensor-elements.

**Multi-variate time-series {{Y}}**

In the description, the sensor-data from the sensor-elements can be represented by a multi-variate time-series {{Y}}, along conventions that the description has discussed above with FIG. 3. As {{Y}} is sensor-data that are not yet processed, {{Y}} can be considered as "raw data". The difference between {{Y}} in FIG. 5A and {{Y}}↓ in FIG. 5B is also distinguished by bold and thin arrows.

**Image**

Image 260 can be a 2D or a 3D arrangement of pixels. As the skilled person is familiar with definition pixels (e.g., locations on the image, pixel data such as color, intensity etc.), the figure omits such well-known details.
Simplified, the gray value of a pixel in image 260 can correlate to the amplitude of an echo arriving. The figure symbolizes gray values by symbols from the keyboard, such as # for "black", * for "grey", and "." for "white". There is a correspondence to the parts of the object.
FIGS. 5A and 5B do not differentiate the images. In an ideal situation (with a method step "enhancing" perfectly performed), both images would be the same.

Display settings are parameters that are related to the way how image 250 is displayed to a user (cf. user 193 in FIG. 1). Display settings can comprise the contrast (e.g., the difference between grey levels), the luminosity (e.g., intensity of pixels on the display), a display resolution (e.g., given by the numbers of pixels per dimension, or simply by the overall number of pixels P), and many others.

Display settings can be modified by the user, by operating user interface elements (e.g., to set the contrast or to set the intensity), by switching hardware (e.g., the display resolution changes from 1280 x 800 with P = 1.024.000 pixels to 1920 x 1080 with P = 2.073.600 pixels), or otherwise.

For the medical application, the practitioner usually has same further display settings available, and the practitioner can communicate display settings via user interface elements to the computer that provides image 260. It is known in the art that images that result from beamforming are further processed by digital filters or the like to highlight particular properties that the practitioner needs to see (e.g., patterns that point to particular medical conditions). In other words, the practitioner may interact with the user interface to select appropriate filtering.

As already mentioned, presenting an image may not be required. A post-processing computer may identify object property the data-structure alone.

**Domains**

Data domains can be differentiated as follows:

- {{ }} or "SIGNAL" stand for the signal-domain (before beamforming), with letters indicating details such as, for example, {{Y}}, {{Y}}↓ and {{Z}}, and

- references 260 or "IMAGE" stand for the image-domain (after beamforming), in other figures also 262, 263.

It is noted that there is no difference in the image (at least here in the figure), no matter if the data comes from a full-number sensor-array (FIG. 5A) or from a sparse sensor-array (FIG. 5B). In other words, insufficiencies in the data availability (i.e., N|s < N|f or data insufficiencies caused otherwise) can be compensated. This is possible due to enhancement in the signal-domain by a trained network (cf. item 233 in FIG. 3), to be explained next.

The domain differentiation is also convenient to explain training criteria.

- training to optimize the data enhancement (cf. the use of a loss function re time-series, cf. step 472-A in FIG. 4, with {{Y}} as the ground truth GT)
- training to optimize the image (cf. the use of a loss function re image, cf. step 472-B in FIG. 4, with an image as the ground truth GT).

**System overview**

FIG. 6 illustrates a block-diagram of a system to obtain an ultrasound image 263|z, 263|f of a physical object (100 cf. FIG. 5) by beamforming. The system comprises a computer with modules 233, 253 to perform a computer-implemented method (to obtain the image of physical object (100 cf. FIG. 5, cf. ) by beamforming.

As FIG. 6 is applicable to monitoring phase \*\*3, the references are given in the \*\*3 notation.

As illustrated on the left side, sensor-array 203|f with N|f sensor-elements 213-1 to 213-N|f can provide multi-variate time-series {{Y}}; and imaging module 253 can convert multi-variate time-series {{Y}} to image 263-f. Image 263-f can be presented to user 193, who is the user of the system (in general) and the user of the computer that implements imaging module 253 (in particular).

Image 263|f can serve as a ground truth GT to train the network module. This will be explained below in step 472-B in method 402, cf. FIG. 8.

However, sensor-array 203|f is not always available, or sensor-array 203|f may not provide {{Y}}, but only {{Y}}.↓.. In other words, it is not expected that - during monitoring in phase \*\*3 - sensor-array 203|f would be available. Instead, it can be expected that sensor-array 203|s is a sparse array (N|s < N|f) with sensor-elements 213-1 to 213-N|s. It provides multi-variate time-series {{Y}}↓ to be pre-processed before it goes into beamforming.

The illustration of sensor-array 203|f as a full sensor-array with selector 223 is convenient, but selector 223 does not have to be implemented as a module that calculates {{Y}}↓ = {{Y}} ⊙ {{M}}. Data selection has been explained above.

As illustrated on the left side, network module 233 is pre-trained network, it receives {{Y}} ↓ (at the input IN) and identifies multi-variate time-series {{Z}} (at the output OUT). {{Z}} is an enhancement to {{Y}} ↓.

Optionally, network module 233 identifies multi-variate time-series Δ{{Z}} (at the output ΔOUT), and combiner module 273 combines {{Y}}↓ and Δ{{Z}} to multi-variate time-series {{Z}}. In that implementation, network module 233 identifies the missing data (i.e., the delta data) only.

Due to enhancing, multi-variate time-series {{Z}} is an approximation of {{Y}}. Imaging module 253 can proceed with beamforming from {{Z}}, resulting in image 263|z (i.e., resulting in the data-structure in general). Image 263-z can be presented to user 263 as well, or alternatively to image 263|f. Image 263|z is an approximation to image 263|f.

The data-structure (be it an image or not) can be further processed (i.e., by post-processing), for speed-of sound estimations or other quantitative imaging modalities.

## Notations

Skilled persons who are familiar with training neural networks are familiar with providing training data is set. For example, a network that should differentiate, dogs from cats would have to be trained not only with a single image of a dog and with a single image of a cat, but with multiple images in sets.

As used herein, using training sets with multiple (i.e., a plurality of) multi-variate time-series $\{\{Y\}\}$ is symbolized by writing SET $\{\{Y\}\}$. The same principle applies to SET $\{\{Y\}\}\downarrow$, also to multiple ground truth "SET GT", and so on.

Training involves to identify network weights at multiple layers of the network ("deep learning") and at multiple nodes within the layers. For simplicity, the description collectively refers to the network weights by writing W2 (during training in phase **2) and W3 (completed training, in phase **3, cf. FIG. 6).

FIG. 7 illustrates a block-diagram of a system to train network 232. The figure is similar to FIG. 6, but the signal flow is different. The figure shows the modules during training phase **2. It further shows two optional loss-calculating modules:

• module 242-S to calculate SIGNAL_LOSS, and

• module 242-I to calculate IMAGE_LOSS.

The concept to have a SIGNAL domain and to have an IMAGE domain has already been introduced above.

Reference sensor-array 202|f has N|f sensor-elements 212-n that provide reference sensor-data, in the form of (a plurality of) multi-variate time-series: SET $\{\{Y\}\}$.

SET $\{\{Y\}\}$ is forwarded (for individual $\{\{Y\}\}$ separately) to the modules as follows:

Selector module 222 applies the above-mentioned selection according to $\{\{Y\}\}\downarrow = \{\{M\}\} \odot \{\{Y\}\}$. In other words, selector module 222 makes the reference sensor-data appear as if they would come from a data-selection.

Network 232 (under training) receives $\{\{Y\}\}\downarrow$ at the input IN (in the mentioned SET) and receives $\{\{Y\}\}$ at the output OUT (also in the mentioned SET). In that sense, network 232 receives data in the selection (e.g. via a bandwidth-limited data link) and receives data without selection, in original full amount.

Optionally, loss-calculating modules 242-S and 242-I provide loss function values. There are two options, to use SIGNAL_LOSS in the signal-domain OR IMAGE_LOSS in the image-domain, or to combine both options.

The figure illustrates modules 242-S and 242-I much simplified. Both modules

• receive ground truth (GT) data (illustrated as GT),

• receive intermediate results from the network (omitted for simplicity), and

• provide loss values that are specific to particular preliminary network weights W2 (the arrows to the left, to network 232), and that are specific to the elements in the SET.

Both loss-calculating modules 242-S and 242-I can use data from modules that would be available from libraries or the like.

For example, imaging module 252 can beamform reference sensor-data $\{\{Y\}\}$ to images 262|f. In principle, there is one image 262|f per $\{\{Y\}\}$. Beamforming is applied to the mentioned data sets: SET $\{\{Y\}\}$ and SET 263|f. Images 262|f can be used by module 242-I to calculate the value IMAGE_LOSS. In other words, IMAGE_LOSS indicates (for each $\{\{Y\}\}$ in the SET and each image 263|f) in the SET how accurate network 232 (under training) performs enhancing with preliminary network weights.

## Improvements in beamforming for training purposes.

Further, imaging module 252 can beamform preliminary data $\{\{Z\}\}$ to hidden images. For simplicity, this beamforming operation is omitted from FIG. 7, but preliminary images go as input to module 242-I.

The description uses the attribute "hidden" in the context of training because these images do not have to be presented to users. Hidden images are to be compared with the GT in module 242-I to calculate the LOSS.

But such hidden images are not calculated only once but in a relatively high number of instances, at least for the following reasons:

• (i) the SET applies, and

• (ii) preliminary network weights W2 lead to different $\{\{Z\}\}$.

However, beamforming (during training, cf. step 442 in FIG. 2 and during monitoring, cf. step 453) requires computational resources, such as computation time. This is a constraint to the training.

Optionally, FIG. 7 writes MODE = (ALPHA, BETA, ...) to symbolize that - for training - beamforming module 252 can operate in different modes. For example, in operation mode ALPHA, module 252 would provide hidden images (to image-loss module 242-I) for the same display settings as module 253 would provide images 263-z, 263-f in the monitoring phase.

As the display settings for module 253 (i.e., in phase **3) can be set by user 193, having a network that has been trained in ALPHA mode is not always available.

As used herein, further Greek letters stand for operation modes for that the operation mode for module 252 would use hidden settings that are different from the display settings.

For example, the modes can be defined in view of the display settings (explained above) as follows:

- In mode BETA, the hidden settings are different from the display settings in terms of pixel numbers. Module 252 provides images in a resolution in that the number of pixels P is in the range from including 40% to including 60% of the number of pixels as for display-images 263. For example, the hidden images have with 1280 x 800 pixels and the display images have 1920 x 1080 pixels.

- In mode GAMMA, module 252 provides speed-of-sound images, whereas the hidden images are ultrasound images with a predetermined resolution. This mode involves the generation of data-structures that have different types. This approach may be advantageous because training steps could be saved. In other words, for the purpose to calculate IMAGE_LOSS, the data-structure for the user (e.g., speed-of-sound) can be different than the data-structure for network-internal training (e.g., ultrasound images).

Instead of operating module 252 in ALPHA mode (that would be optimized to the display settings), operating module 252 in other modes would allow to

- speed up the computation (during training), and

- allow the trained network to execute the enhancing step (cf. 433) and the subsequent beamforming step (cf. 453) for given display settings (and/or for given data-structure settings).

**Computer-implemented method**

FIG. 8 illustrates flow-charts

- for computer-implemented method 402 to train the network, in the training phase **2, on the left side, with the mentioned SET (here symbolized by a repetition line), and

- for computer-implemented method 403 to obtain ultrasound image 252|z, in the monitoring phase **3, on the right side.

Both methods 402 and 403 belong together: training 402 the network in phase **2 is the condition to obtain 403 the image in phase **3 (cf. FIG. 6). Training 402 results in obtaining network weights (W2, W3) for the network (for step enhancing 423 of method 403).
It is possible to perform methods 402 and 403 on separate computers. The figure is simplified in some aspects, but training can be repeated with every new data that becomes available.

**Settings**

For both methods, data-acquisition settings are known in advance. For example, N, T as well as {{M}} are settings that are related to the data that arrives at the computer in both phases (in training **2, FIG. 7 and monitoring **3, FIG. 8).
During monitoring, user 193 can adapt the above-mentioned display settings, and there can be influence to the beamforming, for example by the following:

- The contrast may influence the operation of beamforming module 253.

- The luminosity may not influence the operation, user 193 may simply change the pixel intensity of pixels on the display.

- The display resolution may influence the operation of beamforming module 253 because it would have to provide images in suitable resolution. User 193 may switch the displays. (This is potentially even more sever if the user 193 starts looking at the image on a mobile device (display of smartphone) but continues to look at a larger display of an office computer.

- For the medical application, the user may select filters, or may even change the data-structure being imaged (e.g., from traditional ultrasound image to Doppler image or to speed of sound image).

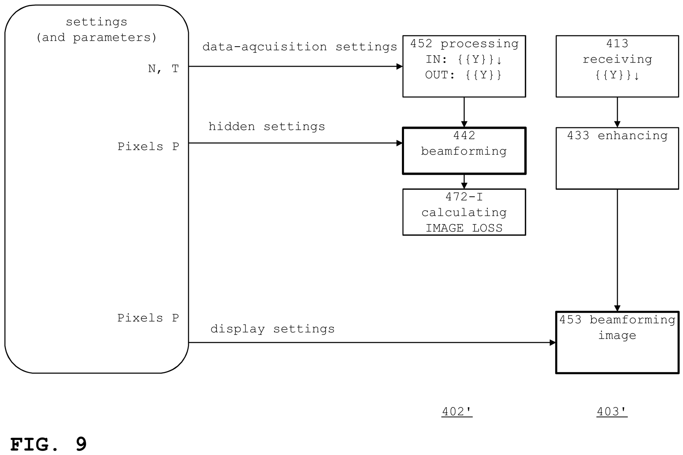

As it will be explained below (cf. FIG. 9), some settings may be different during training and during monitoring. In other words, there is some tolerance available in that so-called hidden settings (that are optionally applied to calculating an image loss) differ from the display settings.

**Training method**

Simplified, training neural networks involves the selection of network weights such that multi-variate time-series {{Y}} (at the input IN of the network) approximates multi-variate time-series {{Z}}. Training 402 is illustrated with

- steps processing 452,

- calculating LOSS 472-A and 472-B (the steps being applied alternatively OR or being applied in combination AND), and

- obtaining network weights 482 (cf. W2 in FIG. 7).

The steps would be performed substantially simultaneously, but in repetitions. For simplicity, some repetitions are omitted. For example, obtaining network weights 482 comprises multiple of previous steps with intermediate network weights W2 to obtain network weights W3 (i.e., final network weights).

Processing 452 comprises to apply the training set SET of multi-variate time-series from the references (i.e., SET{{Y}}, the SET symbolized by step repetitions) to the network (under training, cf. item 232 in FIG. 7).

Calculating LOSS 472-A, 472-B and obtaining network weights 482 belong together, training 402 steps when LOSS reaches pre-defined end conditions (e.g., pre-defined thresholds, no further improvements etc.).

More in detail, in step 452 (of training method 402), the network-under-training receives multi-variate time-series $\{\{Y\}\}\downarrow$ at the input IN and receives multi-variate time-series $\{\{Y\}\}$ at the output OUT. Having data $\{\{Y\}\}$ from a full-number sensor-array (i.e., data with |f) is conceptually the same as knowing the ground truth).

While in step 452, the computer already calculates network weights, at least preliminary ones, method 402 continues with applying training criteria.

The above-mentioned domain differentiation is now explained for steps that involve the calculation of a loss function (or "loss values", to identify the difference to a known ground truth).

- In the signal-domain, multi-variate time-series $\{\{Z\}\}$ that the computer calculates from preliminary network weights may not correspond to time-series $\{\{Y\}\}$ (from full-number sensor-arrays). The SIGNAL_LOSS function (in step 473-A) corresponds to the difference to time-series from full-number sensor-arrays. Beamforming (during training) is not required, it can be bypassed.

- In the image-domain, beamforming 442 (by the beamformer shown in FIG. 6 as item 253, and in FIG. 7 as item 252) can be applied to obtain hidden images, such as image 262|f in FIG. 7. Images are available, (i) as images from full-number sensor-arrays are available (cf. FIG. 7, images 262|f for example), and (ii) as images from network-enhanced time-series $\{\{Z\}\}$, both images can be compared with each other, and the comparison can result in a loss function value IMAGE_LOSS. This is applicable for the mentioned multiple sets.

**Image-domain loss with further details**

Images such as images 263|f (from $\{\{Y\}\}$ and 263|z (from $\{\{Y\}\}\downarrow$ would be suitable for showing to a user (cf. user 193 in FIG. 6). As training (in method 402) should be unsupervised training (i.e., training without involvement of human experts), a comparison of (hidden) images that result from training is not required. Such a comparison would allow calculating IMAGE_LOSS.

Instead, module 242-I can calculate IMAGE_LOSS from hidden images . The hidden have enough data to obtain the image loss, but in resolution (or the like) they may be different from the final images (after step 453, images 263|z) that would be presented to the user.

**Implementation of the network**

The above-mentioned Viñals/Thiran paper explains the architecture for a network (i.e., a convolutional neural network CNN, with deep learning) that can be used to enhance images (not to enhance time-series as described herein). Such a network can also be used as network 232/233 in the function of the enhancing step 433 (cf. FIG. 8).

Loss **weights for training**

The network weights W have been described as the weights that result from training. It is however possible to optionally apply loss weights to IMAGE_LOSS and to SIGNAL_LOSS. These loss weights would be different for both. During training, differences of preliminary data to the ground truths (in the domains SIGNAL and IMAGE) are treated differently.

To simplify training (i.e. in obtaining weights in step 482), the preliminary network weights can be processed with the goal to minimize a single loss value. In such implementation, the single loss value could be calculated as LOSS = IMAGE_LOSS * factor_1 + SIGNAL_LOSS * factor_2

These factors serve as loss weights. Both factors can be the same (i.e., factor_1 = factor_2) and can be set to 1. It is also possible to set factor_1 = 1 and factor_2 = 0.5, or vice versa. Other magnitudes are also possible.

A relation to the above-explained selection is available. As a rule of thumb, the more sparse the data is (i.e., relatively high number of zeros in $\{\{M\}\}$, the more importance has the image domain (i.e., factor_1 > factor_2 as the consequence)

By contrast, when the data is not very sparse (i.e. the 1 in $\{\{M\}\}$ prevail), better results in training can be achieved by the setting the loss weight of the signal domain (i.e., factor_2) higher than the image loss (i.e., factor_2 > factor _1).

**Discussion**

The description continues with a discussion of further aspects. As illustrated by FIG. 3, a multi-variate time-series $\{\{Y\}\}$ is being transmitted from sensor-array 203, either as $\{\{Y\}\}$ or as $\{\{Y\}\}.\downarrow$.. That signals would have to be communicated by a wire connection (e.g., a cable from the probe to a computer) or by radio (e.g., by a wireless link, cf. RADIO in FIG. 1).

A wireless link might not have sufficient capacity (or bandwidth) for $\{\{Y\}\}$, but using $\{\{Y\}\}\downarrow$ instead of $\{\{Y\}\}$

may be an advantage. For the medical application, the medical practitioner could use a wireless probe. This may result in a number of further advantages, for example, cables (from the probe to be computer) would not be required (with some follow-up advantages: no need to disinfect cables before use).

Once the network has been trained (from reference data), it may happen that the sensor-arrays (i.e., transducers) to be used are slightly different from the reference sensor-arrays. The trained networks could potentially be adapted with transfer learning techniques. This approach leverages the existing knowledge from the trained network and fine-tunes them for more specific probes, minimizing the need for extensive retraining.

It is noted that the method steps receiving 413 and beamforming 453 in a sequence that skips enhancing 433 (and combining 473). But that approach would reduce the quality of the image (compared to images 263| f und 263|z).

## Relations between settings

FIG. 9 illustrates a settings configuration module (left side) as well as method 402' (training) and 403' (monitoring) with some steps only.

Data-acquisition settings (e.g., N, T) that mainly defined {{Y}} and {{Y}}$\downarrow$ are related to the data for training (step 452) and for monitoring (step 314).

Hidden settings are relevant for beamforming 442 (that is used to calculate IMAGE_LOSS).

Display settings are relevant for beamforming 453 (to obtain the images to be displayed to the user).

As explained for operation modes ALPHA, BETA etc. for beamforming module 252, the hidden settings and the display settings may be different.

## Generic computer

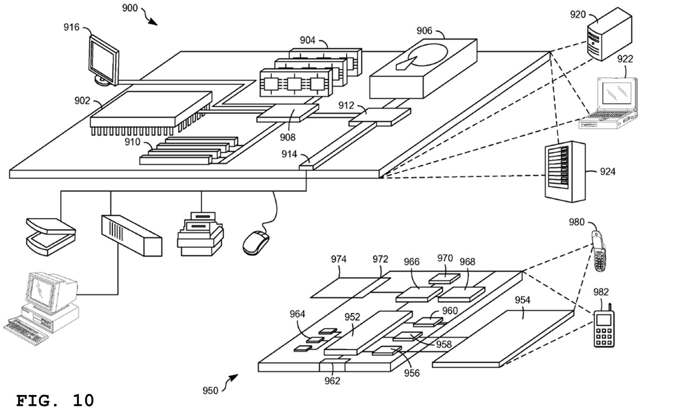

FIG. 10 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Computing device 950 is intended to represent various forms of mobile devices, such as personal digital assistants, cellular telephones, smart phones, and other similar computing devices. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low-speed interface 912 connecting to low-speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high speed interface 908. In other implementations, multiple processors and/or multiple buses may be used, as appropriate, along with multiple memories and types of memory. Also, multiple computing devices 900 may be connected, with each device providing portions of the necessary operations (e.g., as a server bank, a group of blade servers, or a multi-processor system).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high speed controller 908 manages bandwidth-intensive operations for the computing device 900, while the low speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processors. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952, that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the spirit and scope of the invention.

In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. Computer-implemented method (403) to obtain a data-structure (263-z) that is characteristic of a physical object (100), the method involving beamforming, the method (403) comprising:

   a computer (233/253) receiving (413) sensor-data ($\{\{Y\}\}|\downarrow$) from a sensor-array (203|s), wherein the received sensor-data ($\{\{Y\}\}|\downarrow$) is - in spatial and temporal data arrangement - a sub-set of sensor-data ($\{\{Y\}\}$) of a reference sensor-array (202|f) that has N|f ultrasound sensor-elements (212-n|s);

   by a neural network (233) of the computer (233/253) that uses network weights (W3), enhancing (433) the received sensor-data ($\{\{Y\}\}\downarrow$) to enhanced sensor-data ($\{\{Z\}\}$) so that - in spatial and temporal data arrangement - the enhanced sensor-data ($\{\{Z\}\}$) correspond to the sensor-data ($\{\{Y\}\}$) of the reference sensor-array (203|f);

   by an imaging module (253) of the computer (233/253), beamforming (453) the enhanced sensor-data ($\{\{Z\}\}$) to the data-structure (263-z), wherein the neural network (233, 232) has been trained (402) to obtain the network weights (W3), with sensor-data ($\{\{Y\}\}$) from N|f > N|s ultrasound transducer elements (210-n) of the reference sensor-array (202|f) with N|f ultrasound sensor-elements (212-1, 212-N|f).

2. Method (403) according to claim 1, wherein the computer performs the enhancing step (433) such that the received sensor-data ($\{\{Y\}\}\downarrow$) go to an input (IN) of the neural network (233) and the enhanced sensor-data ($\{\{Z\}\}$) are available at an output (OUT) of the neural network (233).

3. Method (403) according to claim 2, wherein the neural network (233, 232) has been trained (402) to obtain the network weights (W3), with the neural network under training (232) having received the sensor-data ($\{\{Y\}\}$) from the reference sensor-array (202|f) at the output (OUT), and
having received selected sensor-data ($\{\{Y\}\}\downarrow$) from N|s < N|f ultrasound transducer elements (110-n) from the reference sensor-array (202|f) through a selector module (222) that applies a selection ($\{\{Y\}\}$, $\{\{Y\}\}\downarrow$).

4. Method (403) according to any of claims 1 to 3, wherein in the step receiving (413), the received sensor-data ($\{\{Y\}\}|\downarrow$) is a sub-set of sensor-data ($\{\{Y\}\}$) of a reference sensor-array (203|f) that is selected from the following:

   a sub-set of sensor-data that is based on a spatial selection that identifies a subset of sensor-elements for that data is to be communicated to the computer (233/253),
   a sub-set of sensor-data that is based on a temporal selection that identifies a subset of time-slots for that data is to be communicated to the computer (233/253), and
   a sub-set of sensor-data that is based on the combination of the spatial selection and the temporal selection.

5. Method (403) according to any of claims 3 and 4, wherein the selector module (222) that applies the selection by the following: performing the receiving step (413) to receive the sensor-data ($\{\{Y\}\}|f$) from the N|f > N|s ultrasound transducer elements (110-n) of the reference sensor-array (202 |f), and applying a selection matrix function ($\{\{M\}\}$).

6. Method (403) according to any of claims 1 to 5, wherein in the step enhancing (433), the computer provides a delta data pattern ($\Delta\{\{Z\}\}$) and wherein in a following step combining (473), the computer combines the delta data pattern ($\Delta\{\{Z\}\}$) to the received sensor-data ($\{\{Y\}\}|\downarrow$).

7. Method according to any of claims 1 to 6, wherein the sensor-data ($\{\{Y\}\}\downarrow$) from the sensor-array (203) with N|s sensor-elements, and the sensor-data ($\{\{Y\}\}|f$) from the reference sensor-array (202|f) is a data in a signal-domain that is represented as multi-variate time-series with the number of variates (N|s) corresponding to the number of sensor-elements (210) that provide data, and with a number of time-slots (T) for measuring an echo, following a wavefront generation.

8. Method according to any of claims 1 to 7, wherein the step enhancing (423) is performed by a network (233, 222) that has a deep learning architecture.

9. Method according to any of claims 1 to 8, wherein the data-structure (263-z) is any of the following a speed-of-sound image, a Doppler image, and an ultrasound image.

10. Method according to any of claims 1 to 9, wherein the neural network (233) has been trained (402) in advance with sensor-data ($\{\{Y\}\}|f$) from N|f > N|s ultrasound sensor-elements (110-n) of the reference sensor-array (202||f) at the output (OUT) and with selected sensor-data (222, $\{\{Y\}\}\downarrow$) at the input (IN).

11. Method according to claim 10, wherein the neural network (233) has been trained (402) with calculating (472-S) a loss function (SIGNAL_LOSS) in the signal-domain with multi-variate time-series ($\{\{Y\}\}$) of the reference sensor-array (202|f) as the ground truth (GT: $\{\{Y\}\}$).

12. Method according to claim 11, wherein the neural network (233) has been trained (402) with further calculating a loss function (IMAGE_LOSS) in an image-domain by beamforming (442) intermediate data to hidden images that are compared to the ground truth of beamformed (252) full-number images (GT:262}f).

13. Method according to claim 12, wherein - to obtain the network weights (482) during training - both the loss function (SIGNAL_LOSS) in the signal-domain and the loss-function (IMAGE_LOSS) in the image-domain are combined such that the loss functions have different loss weights.

14. Computer system (233/253) to obtain a data-structure (263-z) that is characteristic of a physical object (100), wherein the computer system is adapted to execute a method according to any of claims 1 to 13.

15. Computer program product that - when loaded into a memory of a computer and being executed by at least one processor of the computer - cause the computer to perform the steps of the computer-implemented method according to any of claims 1 to 13.

FIG. 1

|   |   |   |   |   | 210-n | 210-(n+x) |   |
|---|---|---|---|---|---|---|---|
| 1 | 2 | 3 | 4 | ... | n | ... | N |

200-1

{{Y}}   {{M}}   {{Y}}↓

220-1

| 1 | 2 | 3 | 4 |   |   |   |   |
|---|---|---|---|---|---|---|---|
|   |   |   |   |   | n |   |   |
|   |   |   |   |   |   |   |   |
|   |   |   |   |   |   |   | N=N1xN2 |

200-2

{{Y}}   {{M}}   {{Y}}↓

220-2

FIG. 2

| | Y1 | Y2 | ... | Yn | ... | YN |
|---|---|---|---|---|---|---|
| time_1 | y | y | y | y | y | y |
| time_2 | y | y | y | y | y | y |
| | y | y | y | y | y | y |
| | y | y | y | y | y | y |
| | y | y | y | y | y | y |
| | y | y | y | y | y | y |
| time_t | y | y | y | y | y | y |
| | y | y | y | y | y | y |
| | y | y | y | y | y | y |
| time_T | y | y | y | y | y | y |

| | M1 | M2 | ... | Mn | ... | MN |
|---|---|---|---|---|---|---|
| time_1 | 1 | 0 | 0 | 1 | 1 | 1 |
| time_2 | 1 | 0 | 0 | 1 | 0 | 0 |
| | 1 | 0 | 0 | 1 | 1 | 1 |
| | 1 | 0 | 0 | 1 | 0 | 0 |
| | 1 | 0 | 0 | 1 | 1 | 1 |
| | 1 | 0 | 0 | 1 | 0 | 0 |
| time_t | 1 | 0 | 0 | 1 | 1 | 1 |
| | 1 | 0 | 0 | 1 | 0 | 0 |
| | 1 | 0 | 0 | 0 | 1 | 1 |
| time_T | 1 | 0 | 0 | 1 | 0 | 0 |

| | Y1 | Y2 | ... | Yn | ... | YN |
|---|---|---|---|---|---|---|
| time_1 | y | | | y | y | y |
| time_2 | y | | | y | | |
| | y | | | y | y | y |
| | y | | | y | | |
| | y | | | y | y | y |
| | y | | | y | | |
| time_t | y | | | y | y | y |
| | y | | | y | | |
| | y | | | | y | y |
| time_T | y | | | y | | |

$$\{\{Y\}\} \qquad \odot \qquad \{\{M\}\}\} \qquad = \qquad \{\{Y\}\}\downarrow$$

FIG. 3

```
**1                    **2                    **3
collecting             training               monitoring
```

N|f

IN N|s

OUT N|f

IN N|s

OUT N|f

$$N|f > N|s$$

**FIG. 4**

FIG. 5A

(A)

100

101

102

(B)

(C)

210-1

210-2

210-n

210-N|s

{{Y}}↓

260
#
#
#
#
#
#
#
*
*
*
*
*
*
.
.
.
.
.
.

200

SIGNAL

IMAGE

**FIG. 5B**

FIG. 6

```
┌─────────────────────────────────────────────────────────────────────────────────┐
│                              202|f reference                                      │
│  ┌─────────┐                      N|f                              ┌─────────┐    │
│  │ 212-1   │      ...                                              │ 212-N|f │    │
│  └─────────┘                                                       └─────────┘    │
└─────────────────────────────────────────────────────────────────────────────────┘
                                      {{Y}}
                              ┌─────────────────┐
                              │      222        │
                              │   selector      │
                              └─────────────────┘
                                    {{Y}}↓
                              ┌─────────────────┐           ┌ ─ ─ ─ ─ ┐
                              │ 232 network IN  │           │  242-S  │
                              │                 │←──────────  SIGNAL_
                              │      W2         │           │  LOSS   │
                              │                 │           └ ─ ─ ─ ─ ┘
                              │                 │           ┌ ─ ─ ─ ─ ┐
                              │      OUT        │←──────────  242-I
                              └─────────────────┘           │ IMAGE_  │
                                                            │  LOSS   │
                                                            └ ─ ─ ─ ─ ┘
┌─────────────────────────────────────────────────────────────────────────────────┐
│ 252 imaging module,beamforming module                                            │
│ MODE = (ALPHA, BETA, ...)                                                         │
└─────────────────────────────────────────────────────────────────────────────────┘
```

GT:{{Y}}}

GT:262|f

**FIG. 7**

training set SET

```
┌─────────────────────────┐        ┌─────────────────────────┐
│      452 processing     │        │           413           │
│       IN: {{Y}}↓        │        │     receiving {{Y}}↓    │
│        OUT: {{Y}}       │        │                         │
└─────────────────────────┘        └─────────────────────────┘

┌──────────────┐ ┌──────────────┐  ┌─────────────────────────┐
│    472-S     │ │     442      │  │      433 enhancing      │
│  calculating │ │  beamforming │  │                         │
│ SIGNAL_LOSS  │ │              │  └─────────────────────────┘
│              │ └──────────────┘
│              │ ┌──────────────┐  ┌ ─ ─ ─ ─ ─ ─ ─ ─ ─ ─ ─ ─ ┐
│              │ │    472-I     │  │     473 combining       │
└──────────────┘ │  calculating │  │                         │
                 │  IMAGE LOSS  │  └ ─ ─ ─ ─ ─ ─ ─ ─ ─ ─ ─ ─ ┘
                 └──────────────┘

┌─────────────────────────┐        ┌─────────────────────────┐
│           482           │        │ 453 beamforming image   │
│     obtaining weights   │        │                         │
│                         │        │                         │
└─────────────────────────┘        └─────────────────────────┘
```

402                                  403

**FIG. 8**

settings
(and parameters)

N, T

Pixels P

Pixels P

data-aqcuisition settings

hidden settings

display settings

| 452 processing |
| IN: {{Y}}↓ |
| OUT: {{Y}} |

| 442 |
| beamforming |

| 472-I |
| calculating |
| IMAGE LOSS |

| 413 |
| receiving |
| {{Y}}↓ |

| 433 enhancing |

| 453 beamforming |
| image |

402'

403'

FIG. 9

FIG. 10

Europäisches
Patentamt

European
Patent Office

Office européen
des brevets

## EUROPEAN SEARCH REPORT

**Application Number**

EP 25 20 8992

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PITMAN WILLIAM M K ET AL: "Branched Convolutional Neural Networks for Receiver Channel Recovery in High-Frame-Rate Sparse-Array Ultrasound Imaging", IEEE TRANSACTIONS ON ULTRASONICS, FERROELECTRICS, AND FREQUENCY CONTROL, IEEE, USA, vol. 71, no. 5, 29 March 2024 (2024-03-29), pages 558-571, XP011969631, ISSN: 0885-3010, DOI: 10.1109/TUFFC.2024.3383660 [retrieved on 2024-04-02] | 1-11,14, 15 | INV. G01S7/52 G01S15/89 G10K11/34 ADD. A61B8/00 |
| A | * the whole document * | 12,13 | |
| | ----- | | |
| A | NAAMA KESSLER ET AL: "Deep-Learning Based Adaptive Ultrasound Imaging from Sub-Nyquist Channel Data", ARXIV.ORG, CORNELL UNIVERSITY LIBRARY, 201 OLIN LIBRARY CORNELL UNIVERSITY ITHACA, NY 14853, 6 August 2020 (2020-08-06), XP081734956, * abstract *; figures 1-3 * * Sections I. and III. * | 1-15 | TECHNICAL FIELDS SEARCHED (IPC) |
| | ----- | | G01S |
| A,D | VIÑALS ET AL: "A KL Divergence-Based Loss for In Vivo Ultrafast Ultrasound Image Enhancement with Deep Learning", J. IMAGING, vol. 9, no. 12, 23 November 2023 (2023-11-23), XP093347238, * the whole document * | 1-15 | G10K A61B |
| | ----- | | |

The present search report has been drawn up for all claims

| Place of search | Date of completion of the search | Examiner |
|---|---|---|
| Munich | 18 December 2025 | Zaneboni, Thomas |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. COHEN** ; **Y. C. ELDAR**. Sparse convolutional beamforming for ultrasound imaging. *IEEE Transactions on Ultrasonics, Ferroelectrics, and Frequency Control*, 2018, vol. 65 (12)

- **ROSER VIÑALS** ; **JEAN-PHILIPPE THIRAN**. *A KL Divergence-Based Loss for In Vivo Ultrafast Ultrasound Image Enhancement with Deep Learning*